# EUROPEAN PATENT APPLICATION

(11) **EP 1 180 367 A1**
(43) Date of publication of application: **20.02.2002**
(21) Application number: 00917435.0
(22) Date of filing: 24.04.2000
(51) Int. Cl.: A61K 31/702, A61K 31/7004, A61P 43/00, A61P 1/00, A23L 1/03, A23L 1/30, A23K 1/16

(54) **INTESTINAL FERMENTATION SULFIDE DEODORIZERS**

(30) Priority: 23.04.1999 JP 11564199
(71) Applicant: Meiji Seika Kaisha, Ltd., Tokyo 104-8002 (JP)
(72) Inventor: INABA, Hiromi Bioscience Labs., Sakado-shi, Saitama 350-0289 (JP); TASHIRO, Yasuhito Meiji Seika Kaisha, Ltd., Kawasaki-shi, Kanagawa 210-0013 (JP); TOKUNAGA, Takahisa Bioscience Labs., Sakado-shi, Saitama 350-0289 (JP); HIRAYAMA, Masao Bioscience Labs., Sakado-shi, Saitama 350-0289 (JP)
(74) Representative: Kyle, Diana
(86) International application number: JP0002666
(87) International publication number: WO0064453

(57) **Abstract**

The present invention provides an agent capable of suppressing the odors of sulfides formed through intra-large-intestinal fermentation, which agent is highly safe and can be widely used in the fields of foods and drugs, and a method of suppressing the same. The agent comprises an indigestible carbohydrate, or a digestible carbohydrate that has been formulated into a dosage form deliverable to the large intestine, wherein the indigestible carbohydrate and the digestible carbohydrate undergo intestinal bacterial fermentation. The method of suppressing the odors of sulfides formed through intra-large-intestinal fermentation in animals comprises administering to an animal an indigestible carbohydrate, or a digestible carbohydrate that has been formulated into a dosage form deliverable to the large intestine, wherein the indigestible carbohydrate and the digestible carbohydrate undergo intestinal bacterial fermentation. The agent and method according to the present invention effectively suppress the odors of sulfides formed through the intra-large-intestinal fermentation, and are highly safe and convenient to use.

## Description

### BACKGROUND OF THE INVENTION

### Fied of the Invention

The present invention relates to an agent for suppressing the odors of sulfides formed through fermentation in the large intestine, and to a method of suppressing the same. More particularly, the present invention relates to an agent for suppressing the odor of methanethiol in animal feces, and to a method of suppressing the same.

### Related Art

Our society is approaching aging society, and we are now confronted with many problems in the care of old people. Unpleasant feelings brought about by a variety of senile smells including the excretal odor may worsen relationships between those people who are caring for the aged and the aged people who are under their care.

Conventionally known means for reducing or preventing the emission of offensive odors include sensory deodorization such as masking; physical means such as adsorption and inclusion; chemical means such as neutralization, oxidation, condensation and addition; and biological deodorization such as the elimination, sterilization or disinfection of malodor-forming bacteria. A variety of deodorizers utilizing these means have already been developed or reported. Upon the development of agents for suppressing the formation of offensive odors, applicable to human beings, attention should be firstly given to safety. For the deodorization of human excreta, there have been proposed extracts of mushrooms (Japanese Laid-Open Patent Publications No. 38358/1993 and No. 238945/1993), phytic acid and phytic acid salts (Japanese Laid-Open Patent Publication No. 120574/1998), molasses made from cane syrup or sugarcane (Japanese Laid-Open Patent Publication No. 151182/1998), and health foods for eliminating the excretal odor, containing as main ingredients dried powders of edible mushrooms such as fukurotake, *hiratake*, *maitake* and *shiitake* (Japanese Laid-Open Patent Publication No. 248154/1997). Further, the use of lactosucrose, an oligosaccharide, has been proposed for the deodorization of pet animals' feces (Japanese Laid-Open Patent Publication No. 115250/1993). However, no reports on the relationship between lactosucrose and the odors of sulfides can be found in the art. Furthermore, there has been reported the relationship between fructooligosaccharide and the production of putrescence (Hidemasa HIDAKA, *Shokuhin Kogyo*, 31, 52-58, 1988, and Makiko KAWAGUCHI, *Bifidobacteria Microflora,* 12, 57-67, 1993). However, the relationship between fructooligosaccharide and methanethiol or dimethyl sulfide has not been reported so far.

### SUMMARY OF THE INVENTION

An object of the present invention is to provide an agent for suppressing the odors of sulfides formed through fermentation in the large intestine, which agent is highly safe and can be widely used in the fields of foods and drugs, and a method of suppressing the same.

We have now found that it is possible to suppress the odors of sulfides, such as methanethiol and dimethyl sulfide, formed through fermentation in the large intestine by delivering carbohydrates that undergo intestinal bacterial fermentation to the large intestine via oral administration. The present invention has been accomplished based on the finding.

An agent for suppressing the odors of sulfides formed through the fermentation in the large intestine according to the present invention comprises an indigestible carbohydrate, or a digestible carbohydrate that has been formulated into a dosage form deliverable to the large intestine, wherein the indigestible carbohydrate and the digestible carbohydrate undergo intestinal bacterial fermentation.

A method of suppressing the odors of sulfides formed through the fermentation in the large intestine in an animal according to the present invention comprises administering to an animal an indigestible carbohydrate, or a digestible carbohydrate that has been formulated into a dosage form deliverable to the large intestine, wherein the indigestible carbohydrate and the digestible carbohydrate undergo intestinal bacterial fermentation.

When the agent according to the present invention is administered to an animal, the indigestible or digestible carbohydrate reaches the large intestine and suppresses the formation of sulfides (e.g., methanethiol, dimethyl sulfide, etc.), main components of the odor of animal feces, so that the feces do not so much emit the odors of sulfides. Further, the agent and method according to the present invention use those indigestible oligosaccharides or digestible carbohydrates that have been commonly used in foods or as food additives. Furthermore, the agent of the invention can be orally administered to animals. Thus, the agent and method according to the present invention are highly safe and convenient to use.

Although the present invention is not restricted to the following theory, it is thought that the odors of sulfides formed through the fermentation in the large intestine are suppressed by the following mechanism: intestinal bacteria ferment the carbohydrate that has reached the large intestine in preference to sulfur-containing amino acids and, as a result, the metabolism from the sulfur-containing amino acids to the odors of sulfides is suppressed.

### DETAILED DESCRIPTION OF THE INVENTION

In the present invention, the term "formed through fermentation in the large intestine" means to be undergone fermentation caused by intestinal bacteria in the large intestine. The term "indigestible" herein means to undergo substantially no digestion, absorption, or metabolism in the stomach and small intestine and to be delivered to the large intestine.

Indigestible carbohydrates and digestible carbohydrates for use in the present invention are characterized by undergoing intestinal bacterial fermentation. These carbohydrates may be selected from those ones that have conventionally been used as ingredients of foods or as food additives.

In the present invention, it is essential that the carbohydrate reach the large intestine. Therefore, although it is possible to use indigestible carbohydrates as they are, it is necessary, before use, to formulate digestible carbohydrates into dosage forms deliverable to the large intestine. It is preferable to coat digestible carbohydrates with coating agents that are disintegrable in the large intestine to make them disintegrable in the same. Known coating methods useful for this purpose are as follows: a method in which a digestible carbohydrate is coated to form a capsule around it (e.g., Japanese Laid-Open Patent Publication No. 41422/1992); a method in which glucose is coated with chitosan and a specific polymer (Japanese Laid-Open Patent Publication No. 34927/1991); and a method in which an active component is multi-coated with a layer of chitosan and that of a material resistant to acid in the stomach (Japanese Patent Application No. 73599/1997).

Examples of indigestible carbohydrates that undergo intestinal bacterial fermentation include indigestible oligosaccharides such as fructooligosaccharide, galactooligosaccharide, xylooligosaccharide and raffinose, and inulins obtainable from chicory and Jerusalem artichoke. Of these, fructooligosaccharide and its constitutive saccharides are preferred.

Fructooligosaccharide is an oligosaccharide widely distributed in nature; it is a mixture of saccharides composed of the fructose residues of sucrose to which 1 to 3 fructose molecules are connected via β2-1 bond. Nistose, kestose and fructofuranosylnystose, constitutive saccharides of fructooligosaccharide, can be used even singly as an agent for suppressing the odors of sulfides according to the present invention. Since fructooligosaccharide has excellent physiological effects such as the effect of promoting the growth of Bifidobacterium in the intestine, the effect of normalizing the intestinal conditions, the effect of preventing tooth decay and the effect of promoting mineral absorption, they are used as new functional materials in the fields of foods and feeds. In addition, they have been approved as "Food for Specific Health Use", so that they are, of course, very safe materials.

Examples of digestible carbohydrates that undergo intestinal bacterial fermentation include glucose, fructose, sucrose, trehalose, maltose, maltooligosaccharide and isomaltooligosaccharide.

The agent according to the present invention may comprise the carbohydrate either singly or in combination with other deodorizing components. As long as carbohydrates are deliverable to the large intestine, the agent according to the present invention may take any dosage form. Namely, in the present invention, indigestible carbohydrates in the form of liquid or powder can be used either. Indigestible carbohydrates can also be used in combination with foods or pharmaceutical materials. It is also possible to use digestible carbohydrates after coating them, together with other deodorizing components, with coating agents disintegrable in the large intestine. The agent according to the present invention may be administered via various routes, such as rectal administration, besides oral administration.

The carbohydrate content of the agent according to the present invention may be determined depending upon the properties of a food, beverage or medicine into which the agent will be incorporated, preference, the desired degree of sweetness, profitability, and the like. It may be 20% by weight or less, preferably about 0.1 to 10% by weight. The carbohydrate may be formulated in any proper stage of the production of the agent.

Since carbohydrates have sweetness of good quality, they can also be used as alternatives or partial alternatives of sweetening agents. Specifically, their uses for this purpose include seasonings for home use, table sugar, and sweeteners for coffee or tea. Moreover, it is also possible to apply the agent according to the present invention to sugar-free foods by replacing sweetening agents with highly pure indigestible carbohydrates (e.g., fructooligosaccharide, trade name "Meioligo P", manufactured by Meiji Seika Kaisha, Ltd., Japan, the purity = 95% or more). It is of course possible to add the agent according to the present invention to feeds for pet or domestic animals. Thus, the agent and method according to the present invention are applicable not only to a variety of foods and beverages, drugs and quasi-drugs, but also to feeds for pet or domestic animals, and the like.

### EXAMPLES

The present invention is further illustrated by the following Examples that are not intended as a limitation of the invention.

Throughout the examples, "%" means "% by weight", unless otherwise specified.

The determination of methanethiol was conducted by gas chromatography. Specifically, a gas chromatograph GC-9AM manufactured by Shimadzu Corp, Japan, was used for the determination together with 25% β,β-Oxydipropionitrile Chromosorb W AW-DMCS (60-80 meshes, 3.1 m × 3.2 mm) as a column, 50 ml/min of nitrogen as a carrier gas, and FPD as a detector. The determination was conducted under the conditions of an inlet temperature of 150°C, an evaporation chamber temperature of 180°C, a detector temperature of 180°C, and a column temperature of 70°C.

"Meioligo P" manufactured by Meiji Seika Kaisha, Ltd., Japan was used for fructooligosaccharide.

### Example 1: Effect of Fructooligosaccharide on the Formation of Sulfides in Incubation of Feces

Fresh human feces were suspended in a 20 mM phosphoric acid - 110 mM bicarbonate buffer solution having a pH of 6.86 [*Barry J-L*., *British J*. *Nutrition,* 74, 303-322] that had been saturated with carbon dioxide and heated to a temperature of 37°C, thereby obtaining a suspension having a feces content of 20.0%. This suspension was filtered through six sheets of gauze laid one over another, and the filtrate was used as an inoculum in the subsequent step. 2.5 ml of the inoculum was placed in each one of three 19-ml vials for gas chromatography, and the vials were airtightly sealed under anaerobic condition. Before starting incubation, 0.5 ml of the above-described buffer solution was added to the first vial containing the inoculum (Control Group, final concentrations: methionine 0.0%, fructooligosaccharide 0.0%); a solution of methionine, the precursor of methanethiol, was added to the second vial (Methionine Group, final concentrations: methionine 0.005%, fructooligosaccharide 0.0%); and a solution mixture of methionine and fructooligosaccharide was added to the third vial (Fructooligosaccharide Group, final concentrations: methionine 0.005%, fructooligosaccharide 1.0%). These vials were subjected to mild shake incubation at 37°C for 3 hours and 18 hours. Immediately after the incubation, the concentration of methyl mercaptan in the headspace of each vial was determined by gas chromatography.

By the use of fresh feces of three male adults, three inoculums were respectively prepared in the above-described manner, and the aforementioned experiment was conducted by using these inoculums. The results were as shown Table 1. As is clear from the results obtained after the 3-hour incubation, the methanethiol concentrations in all of the samples of Fructooligosaccharide Group were-below the limit of detection; thus the effect of suppressing the formation of sulfides was notably obtained by the ingestion of fructooligosaccharide. Moreover, this effect was confirmed even after the 18-hour incubation.

**Table 1:**

| Effect of Fructooligosaccharide on Suppression of the Formation of Sulfides | | | | |
|---|---|---|---|---|
| | | Concentrationn of Methanethiol (ppm) | | |
| | Volunteer Code | Control Group | Methionine Group | Fructooligosaccharide Group |
| After 3-hour Incubation | A | 0.11 | 0.56 | ND |
| | B | 0.25 | 1.57 | ND |
| | C | 0.01 | 0.01 | ND |
| After 18-hour Incubation | A | 0.66 | 1.91 | 0.88 |
| | B | 0.31 | 1.70 | 0.26 |
| | C | 1.47 | 1.68 | 1.13 |

### Example 2: Effect of Glucose on the Formation of Sulfides in Tncubation of Feces

An inoculum was prepared in the same manner as in Example 1, and samples of Control Group, Methionine Group and Fructooligosaccharide Group were prepared as described in Example 1. In addition, a solution mixture of methionine and glucose was added to the fourth vial (glucose Group, final concentrations: methionine 0.005%, glucose 1.0%), the concentration of glucose being the same as that of the fructooligosaccharide in Example 1. After conducting incubation for 2.5 hours, the concentration of methanethiol in the headspace of each vial was determined. The results were as shown in Table 2. The formation of methanethiol was greatly suppressed both by the addition of fructooligosaccharide and by the addition of glucose. It is thus confirmed that the supply of the indigestible carbohydrate or digestible carbohydrate to the large intestine is very effective for suppressing the formation of sulfides.

**Table 2:**

| Effect of Glucose on Suppression of the Formation of Sulfides | | | | |
|---|---|---|---|---|
| | Concentration of Methanethiol (ppm) | | | |
| Volunteer Code | Control Group | Methionine Group | Fructooligosaccharide Group | Glucose Group |
| Y | 0.14 | 0.41 | 0.03 | 0.07 |

### Example 3: Effect of Fructooligosaccharide on the Odors of Sulfides in Incubation of Feces

An inoculum was prepared from human fresh feces in the same manner as in Example 1. Samples of Control Group, Meat Extract Group (final concentration: meat extract 0.01%), Fructooligosaccharide Group (final concentration: fructooligosaccharide 1.0%), and Meat Extract + Fructooligosaccharide Group (final concentrations: meat extract 0.01%, fructooligosaccharide 1.0%) were respectively prepared as described in Example 1, and then subjected to incubation. It is noted that the meat extract was added as the source of an offensive odor. After the incubation was completed, the gas in the headspace of each vial was introduced into a smelling bag (manufactured by GL Sciences Inc., Japan), and the gases in the smelling bags were diluted to the same degree. The odors of the gases were organoleptically evaluated. This organoleptic test was carried out by three professional panelists. The evaluation was carried out by ranking the odors of the gases in comparison with those of the gases of Control Group in accordance with the following criteria: -2 (the odor is almost nil), -1 (the odor is hardly perceived), ±0 (the odor is easily perceived), +1 (the odor is strong), and +2 (the odor is terribly strong).

As shown in Table 3, the meat extract increased the degree of an offensive odor (cf. Control Group and Meat Extract Group), while the degree of the offensive odor was lowered by the addition of fructooligosaccharide (cf. Fructooligosaccharide Group and Meat Extract + Fructooligosaccharide Group). These results of the organoleptic test agree with those of the instrumental analysis conducted in Example 1.

**Table 3:**

| Results of Organoleptic Test on Headspace Gases | | | | |
|---|---|---|---|---|
| Panelist Code | Control Group | Meat Extract Group | Fructooligo Saccharide Group | Meat Extract + Fructooligosaccharide Group |
| A | ±0 | +1 | -1 | -1 |
| B | ±0 | +2 | -1 | -1 |
| C | ±0 | -1 | -2 | -2 |

### Example 4: Effect of Fructooligosaccharide on the Odors of Sulfides in Human Feces

3 g of fructooligosaccharide was given to 14 women students (age: 20 to 24 years) once a day at the time of supper, and this was repeated for 14 consecutive days. The feces of the students were anaerobically collected on day 14, and stored at a low temperature until the following analysis was conducted. 0.5 g of the feces of each student was weighed into a 19-ml glass vial, and the vials were sealed airtightly. These vials were then heated at 37°C for 30 minutes, and the headspace gases were subjected to the determination of the concentration of methanethiol. It is noted that, prior to this test, the concentration of methanethiol in the feces of each student was measured for three days, and the average value was obtained as the concentration in the fructooligosaccharide-non-ingestion period.

As shown in Fig. 4, methanethiol was detected in the feces of 5 out of the 14 students collected in the fructooligosaccharide-non-ingestion period, while the methyl mercaptan concentrations in the feces samples of the other 9 students in this period were below the limit of detection. On the other hand, after the ingestion of fructooligosaccharide, the number of feces samples in which methanethiol was detected was decreased to 3. Moreover, the average of the methyl mercaptan concentrations in these 3 samples was as low as approximately 1/3 of the average of the concentrations in the non-ingestion period. These results demonstrate that the formation of sulfides can be suppressed by the ingestion of fructooligosaccharide that can reach the large intestine.

**Table 4:**

| Effect of Fructooligosaccharide on Suppression of the Formation of Sulfides in Human Beings | | | | | | |
|---|---|---|---|---|---|---|
| | Concentration of Methyl Mercaptan in Feces [µg/100 ml] (average value, n=3) | | | | | |
| | Volunteer Code | | | | | |
| | A | B | C | D | E | Average |
| Fructooligosaccha ride Non-Ingestion period | 0.01 | 0.12 | 4.65 | 10.80 | 0.25 | 3.17 |
| Ingestion period | ND | ND | 0.07 | 4.86 | 0.12 | 1.01* |
| ND: Below the limit of determination | | | | | | |

| | | | | | | |
|---|---|---|---|---|---|---|
| *: The average value when ND is taken as 0.00. | | | | | | |

### Example 5: Effect of glucose Disintegrable in the Large Intestine on the Odors of Sulfides in Feces of Human Beings

### (1) Process of Producing Glucose Disintegrable in the Large Intestine

Cellulose and glucose were sprinkled on a nucleus containing cellulose and lactose, and then dried to give particles containing 40% of glucose, having a diameter of 4.7 mm. A 3.5% aqueous chitosan solution containing 0.8% of glycerin was sprayed over the above-obtained particles, and dried, thereby obtaining particles coated with a chitosan layer having a thickness of approximately 130 µm. Subsequently, 5.9% Zein containing 0.8% of glycerin was dissolved in an 80% aqueous ethanol solution, and this solution was dried by spraying over the above particles to give glucose disintegrable in the large intestine.

### (2) Effect of Glucose Disintegrable in the Large Intestine on the Formation of Sulfides in Feces of Human Beings

3 g of the glucose disintegrable in the large intestine was given to 7 healthy male adults once a day at the time of supper, and this was repeated for 7 consecutive days. The feces of the subjects on day 7 were anaerobically collected, and the methanethiol concentrations in the feces of the subjects were determined in the same manner as in Example 4. It is noted that, prior to this test, the concentration of methanethiol in the feces of each subject was measured.

As a result, before the ingestion of the glucose disintegrable in the large intestine to the subjects, methanethiol was detected in the feces of 4 out of the 7 subject, while the methanethiol concentrations in the feces of 3 out of the 7 subjects were below the limit of detection. On the other hand, after the ingestion of the glucose disintegrable in the large intestine, methanethiol was found in the feces of only 2 out of the 7 subjects. These results demonstrate that the ingestion of the glucose disintegrable in the large intestine is highly effective for suppressing the formation of methanethiol.

### Comparative Example: Test for Evaluating the Effect of Fructooligosaccharide and Mushroom Extract on Suppression of the Formation of Methanethiol.

An inoculum was prepared in the same manner as in Example 1, and samples of Control Group and Fructooligosaccharide Group were prepared as described in Example 1. In addition, a mushroom extract ("Bio-M Bx50", a concentrated champignon extract manufactured by Rikomu) was added to the vial so that its final concentration would be Bx1.0 (Mushroom Extract Group). The concentration of methanethiol in the headspace of each vial was determined after 3- and 18-hour incubation.

As shown in Table 5, the initial formation of methanethiol (after the 3-hour incubation) in the sample of Fructooligosaccharide Group was suppressed more greatly than that in the sample of Control Group, and this effect was confirmed even after the 18-hour incubation. It is noted that the concentration of methanethiol in the sample of Fructooligosaccharide Group after the 18-hour incubation is only 21% of that in the sample of Control Group. On the other hand, the initial formation of methanethiol was also suppressed in the sample of Mushroom Extract Group, but this effect is not so remarkable as that in the sample of Fructooligosaccharide Group.

**Table 5:**

| Comparison between the Effect of Fructooligosaccharide and that of Mushroom Extract | | |
|---|---|---|
| Group | Concentration of Methanethiol (ppm) After-3hour Incubation After 18-hour Incubation | |
| Control | 0.597 | 1.393 |
| Fructooligo Saccharide | ND | 0.293 |
| Mushroom Extract | 0.177 | - |
| ND: Below the limit of determination | | |
| -: Not tested | | |

## Claims

1. An agent for suppressing the odors of sulfides formed through fermentation in the large intestine, comprising an indigestible carbohydrate, or a digestible carbohydrate that has been formulated into a dosage form deliverable to the large intestine, wherein the indigestible carbohydrate and the digestible carbohydrate undergo intestinal bacterial fermentation.

2. The agent for suppressing the odors of sulfides according to claim 1, wherein the indigestible carbohydrate is fructooligosaccharide, nistose, kestose or fructofuranosylnistose.

3. The agent for suppressing the odors of sulfides according to claim 1, wherein the digestible carbohydrate has been coated to make it disintegrable in the large intestine.

4. The agent for suppressing the odors of sulfides according to claim 1 or 3, wherein the digestible carbohydrate is glucose.

5. The agent for suppressing the odors of sulfides according to any one of claims 1 to 4, capable of suppressing the odors of sulfides in feces.

6. The agent for suppressing the odors of sulfides according to any one of claims 1 to 5, wherein the sulfides include methanethiol.

7. The agent for suppressing the odors of sulfides according to any one of claims 1 to 6, which is in a formulation suitable for oral administration.

8. A method of suppressing the odors of sulfides formed through fermentation in the large intestine in an animal, comprising administering to an animal an indigestible carbohydrate, or a digestible carbohydrate that has been formulated into a dosage form deliverable to the large intestine, wherein the indigestible carbohydrate and the digestible carbohydrate undergo intestinal bacterial fermentation.

9. The method according to claim 8, wherein the indigestible carbohydrate is fructooligosaccharide, nistose, kestose or fructofuranosylnistose.

10. The method according to claim 8, wherein the digestible carbohydrate has been coated to make it disintegrable in the large intestine.

11. The method according to claim 8 or 10, wherein the digestible carbohydrate is glucose.

12. The method according to any one of claims 8 to 11, wherein the odors of sulfides in feces are suppressed.

13. The method according to any one of claims 8 to 12, wherein the sulfides include methanethiol.

14. The method according to any one of claims 8 to 13, wherein the indigestible carbohydrate, or the digestible carbohydrate that has been formulated into a dosage form deliverable to the large intestine, is orally administered.

15. The method according to any one of claims 8 to 14, wherein the animal is a human.
